(19) Europäisches Patentamt
European Patent Office
Office européen des brevets

(11) Veröffentlichungsnummer: **0 603 696 A1**

# (12) EUROPÄISCHE PATENTANMELDUNG

(21) Anmeldenummer: **93120038.0**

(22) Anmeldetag: **11.12.93**

(51) Int. Cl.$^5$: **C07D 471/04**, A01N 43/90, //(C07D471/04,221:00,221:00)

(30) Priorität: **21.12.92 DE 4243286**

(43) Veröffentlichungstag der Anmeldung: **29.06.94 Patentblatt 94/26**

(84) Benannte Vertragsstaaten: **AT BE CH DE DK ES FR GB GR IE IT LI NL PT SE**

(71) Anmelder: **BASF Aktiengesellschaft**
**Carl-Bosch-Strasse 38**
**D-67063 Ludwigshafen(DE)**

(72) Erfinder: **Mueller, Thomas, Dr.**
**Bergstrasse 19**
**D-67258 Hessheim(DE)**
Erfinder: **Ammermann, Eberhard, Dr.**
**Von-Gagern-Strasse 2**
**D-64646 Heppenheim(DE)**
Erfinder: **Lorenz, Gisela, Dr.**
**Erlenweg 13**
**D-67434 Neustadt(DE)**
Erfinder: **Harries, Volker, Dr.**
**Immengaertenweg 29e**
**D-67227 Frankenthal(DE)**
Erfinder: **Risch, Nikolaus, Prof. Dr.**
**Am Liemer Turmhof 2**
**D-32657 Lemgo(DE)**
Erfinder: **Westerwelle, Ulrich**
**Apfelstrasse 141a**
**D-33611 Bielefeld(DE)**

(54) **Verwendung von substituierten Dipyridin-Derivaten zur Bekämpfung von Schädlingen.**

(57) Verwendung von substituierten Dipyridin-Derivaten der allgemeinen Formel I,

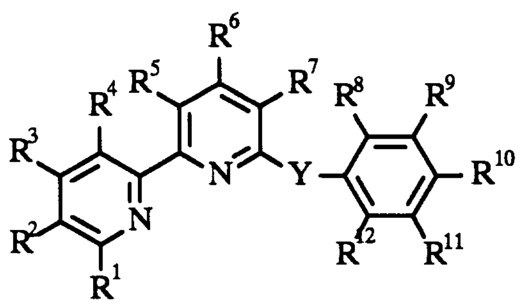

I

worin

| | |
|---|---|
| $R^1$,$R^2$,$R^3$ = | unabhängig voneinander Wasserstoff, Alkyl, Phenyl, Halogen, |
| $R^4$ und $R^5$ | zusammen eine Polymethylenkette der Formel $-(CH_2)_m-$, |
| $R^6$ = | Wasserstoff, |
| $R^7$ = | Wasserstoff, Alkyl, Phenylalkyl, Phenyl, |
| $R^8$ = | Wasserstoff, Alkyl, Alkoxy, Haloalkyl, Halogen oder |
| $R^7$ und $R^8$ | können zusätzlich zusammen eine Polymethylenkette der Formel $-(CH_2)_n-$ bilden, |
| $R^9$,$R^{10}$,$R^{11}$, $R^{12}$ = | unabhängig voneinander Wasserstoff, Alkyl, Alkoxy, Haloalkyl, Phenyl, Halogen, Nitro, |
| Y = | eine Einfachbindung oder $-CH_2-$, |

bedeuten, sowie deren pflanzenverträglichen Säureadditionssalze und Metallsalzkomplexe zur Bekämpfung von Schädlingen und Verbindungen der Formel I.

EP 0 603 696 A1

Die vorliegende Erfindung betrifft die Verwendung von teilweise bekannten, substituierten Dipyridin-Derivaten zur Bekämpfung von Schädlingen, insbesondere Pilzen oder Nematoden, sowie neue substituierte Dipyridin-Derivate und Verfahren zu ihrer Herstellung.

Es sind substituierte Dipyridin-Derivate bekannten (Chem. Ber. 124 (1991) 571); über ihre Wirksamkeit im Pflanzenschutz ist jedoch nichts bekannt.

Es wurde nun gefunden, daß die teilweise bekannten, substituierten Dipyridin-Derivaten der allgemeinen Formel I,

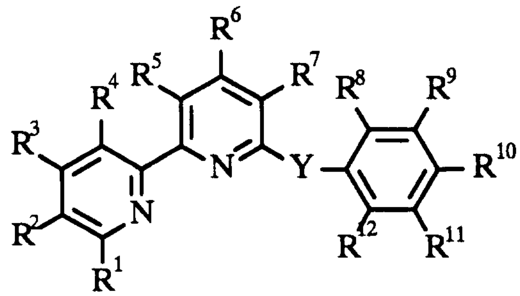

I

worin

| | |
|---|---|
| $R^1$,$R^2$,$R^3$ = | unabhängig voneinander Wasserstoff, $C_1$-$C_4$-Alkyl, Phenyl, Halogen, |
| $R^4$ und $R^5$ | zusammen eine Polymethylenkette der Formel - $(CH_2)_m$-mit m = 1 bis 4 bilden, |
| $R^6$ = | Wasserstoff, |
| $R^7$ = | Wasserstoff, $C_1$-$C_4$-Alkyl, Phenyl-$C_1$-$C_2$-alkyl, Phenyl, wobei die beiden letztgenannten Reste im Phenylteil bis zu dreifach durch Halogen, $C_1$-$C_4$-Alkyl, $C_1$-$C_4$-Alkoxy oder $C_1$-$C_4$-Haloalkyl substituiert sein können, |
| $R^8$ = | Wasserstoff, $C_1$-$C_4$-Alkyl, $C_1$-$C_4$-Alkoxy, $C_1$-$C_4$-Haloalkyl, Halogen oder |
| $R^7$ und $R^8$ | können, für den Fall Y = Einfachbindung, zusätzlich zusammen eine Polymethylenkette der Formel - $(CH_2)_n$ - mit n = 1 bis 4 bilden, |
| $R^9$,$R^{10}$,$R^{11}$, $R^{12}$ = | unabhängig voneinander Wasserstoff, $C_1$-$C_4$-Alkyl, $C_1$-$C_4$-Alkoxy, $C_1$-$C_4$-Haloalkyl, Phenyl, Halogen, Nitro, |
| Y = | eine Einfachbindung oder -$CH_2$-, |

bedeuten, sowie deren pflanzenverträglichen Säureadditionssalze und Metallsalzkomplexe zur Bekämpfung von Schädlingen, insbesondere Pilzen und Nematoden, geeignet sind.

Als Säureadditionssalze eignen sich die pflanzenverträglichen Salze von solchen Säuren, die die biologische Wirkung von I nicht beeinträchtigen, z. B. die Iodide, Chloride, Bromide, Sulfate, Dodecylsulfate, Nitrate, Carbonate, Phosphate, Borate, Formiate, Acetate, Propionate, Benzoate, Oxalate, Naphthalinsulfonate, Dodecylbenzolsulfonate, Lactate, Citrate und die Salze mit dem Anion des Saccharins.

Als Metallkomplexe kommen die Komplexe des Calciums, Magnesiums, Kupfers, Zinks, Zinns, Mangans, Eisens, Kobalts oder Nickels in Betracht. Vorzugsweise stellt man die Komplexe aus den freien Basen I und den mineralsauren Salzen, beispielsweise den Chloriden oder Sulfaten, der Metalle her.

Ein weiterer Gegenstand der vorliegenden Erfindung sind neue, substituierte Dipyridin-Derivate der Formel I, worin

| | |
|---|---|
| $R^1$,$R^2$,$R^3$ = | unabhängig voneinander Wasserstoff, $C_1$-$C_4$-Alkyl, wie Methyl, Ethyl, Propyl, 1-Methylethyl, Butyl, 1-Methylpropyl, 2-Methylpropyl und 1,1-Dimethylethyl, insbesondere Methyl und Ethyl, Phenyl, Halogen, wie Fluor, Chlor, Brom, Jod, insbesondere Chlor und Brom, |
| $R^4$ und $R^5$ = | zusammen eine Polymethylenkette der Formel - $(CH_2)_m$-mit m = 1 bis 4 bilden, vorzugsweise m = 2, |
| $R^6$ = | Wasserstoff, |
| $R^7$ = | Wasserstoff, $C_1$-$C_4$-Alkyl, wie Methyl, Ethyl, Propyl, 1-Methylethyl, Butyl, 1-Methylpropyl, 2-Methylpropyl und 1,1-Dimethylethyl, insbesondere Methyl und Ethyl, Phenyl-$C_1$-$C_2$-alkyl, insbesondere Benzyl, 1-Phenylethyl, 2-Phenylethyl, Phenyl, wobei die beiden letztgenannten Reste im Phenylteil bis zu dreifach durch Halogen, wie Fluor, Chlor, Brom, Jod, insbesondere Fluor und Chlor, $C_1$-$C_4$-Alkyl, wie Methyl, Ethyl, Propyl, 1-Methylethyl, Butyl, 1-Methylpropyl, 2-Methylpropyl und 1,1-Dimethylethyl, insbesondere Methyl, 1-Methylethyl und 1,1-Dimethylethyl, $C_1$-$C_4$-Alkoxy, wie Methoxy, Ethoxy, Propyloxy, 1-Methylethoxy, Butyloxy, 1-Me- |

thylpropyloxy, 2-Methylpropyloxy und 1,1-Dimethylethoxy, insbesondere Methoxy, $C_1$-$C_4$-Haloalkyl, wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Di-fluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl, substituiert sein können,

$R^8$ = Wasserstoff, $C_1$-$C_4$-Alkyl, wie Methyl, Ethyl, Propyl, 1-Methylethyl, Butyl, 1-Methylpropyl, 2-Methylpropyl und 1,1-Dimethylethyl, insbesondere Methyl, $C_1$-$C_4$-Alkoxy, wie Methoxy, Ethoxy, Propyloxy, 1-Methylethoxy, Butyloxy, 1-Methylpropyloxy, 2-Methylpropyloxy und 1,1-Dimethylethoxy, insbesondere Methoxy, $C_1$-$C_4$-Haloalkyl, wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluor-ethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl, Halogen, wie Fluor, Chlor, Brom, Jod, insbesondere Fluor und Chlor, oder

$R^7$ und $R^8$ können, für den Fall Y = Einfachbindung, zusätzlich zusammen eine Polymethylenkette der Formel $-(CH_2)_n-$ mit n = 1 bis 4 bilden, vorzugsweise n = 2,

$R^9$,$R^{10}$,$R^{11}$, $R^{12}$ = unabhängig voneinander Wasserstoff, $C_1$-$C_4$-Alkyl, wie Methyl, Ethyl, Propyl, 1-Methylethyl, Butyl, 1-Methylpropyl, 2-Methylpropyl und 1,1-Dimethylethyl, insbesondere Methyl, $C_1$-$C_4$-Alkoxy, wie Methoxy, Ethoxy, Propyloxy, 1-Methylethoxy, Butyloxy, 1-Methylpropyloxy, 2-Methylpropyloxy und 1,1-Dimethylethoxy, insbesondere Methoxy, $C_1$-$C_4$-Haloalkyl, wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2-Chlor-2,2-difluorethyl, 2,2-Dichlor-2-fluorethyl, 2,2,2-Trichlorethyl und Pentafluorethyl, insbesondere Trifluormethyl, Phenyl, Halogen, wie Fluor, Chlor, Brom, Jod, insbesondere Fluor und Chlor, Nitro,

Y = eine Einfachbindung oder $-CH_2-$,

bedeuten, sowie deren pflanzenverträgliche Säureadditionssalze und Metallsalzkomplexe, ausgenommen die Verbindungen der Formel I, in der die Reste $R^1$, $R^2$, $R^3$, $R^6$, $R^9$, $R^{10}$, $R^{11}$ und $R^{12}$ Wasserstoff und Y eine Einfachbindung bedeuten und $R^4$, $R^5$, $R^7$ und $R^8$ die folgenden Bedeutungen haben:

| $R^4$, $R^5$ | $R^7$ | $R^8$ |
|---|---|---|
| $-CH_2-$ | $-CH_2-$ | |
| $-CH_2-$ | $-(CH_2)_2-$ | |
| $-(CH_2)_2-$ | $-CH_2-$ | |
| $-(CH_2)_2-$ | $-(CH_2)_2-$ | |
| $-CH_2-$ | $CH_3$ | H |
| $-CH_2-$ | Ph | H |
| $-(CH_2)_2-$ | Ph | H |

Ein weiterer Gegenstand der vorliegenden Erfindung ist ein Verfahren zur Herstellung der substituierten Dipyridin-Derivate I gemäß Anspruch 2, in dem man eine Verbindung der Formel II,

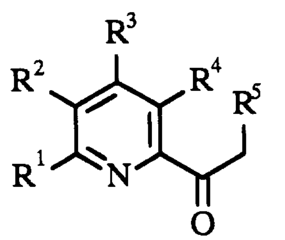

II

worin $R^1$ bis $R^5$ die Bedeutung wie in Formel I gemäß Anspruch 1 besitzen, mit einer Verbindung der Formel III,

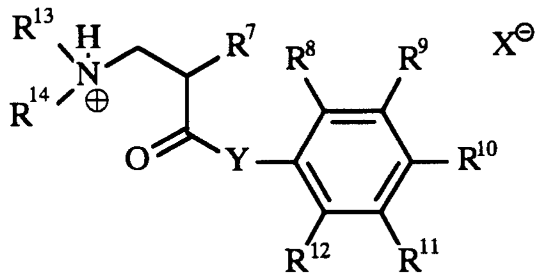

III

worin $R^7$ bis $R^{12}$ und Y die Bedeutung wie in Formel I gemäß Anspruch 1 besitzen, X das Anion einer organischen oder anorganischen Säure, wie das Chlorid-, Bromid-, Sulfat-, Perchlorat- oder Acetat-Ion, und $R^{13}$ und $R^{14}$ unabhängig voneinander $C_1$-$C_4$-Alkyl, wie Methyl, Ethyl, Propyl, 1-Methylethyl, Butyl, 1-Methylpropyl, 2-Methylpropyl und 1,1-Dimethylethyl, insbesondere Methyl, oder $C_5$-$C_7$-Cycloalkyl, wie Cyclopentyl, Cyclohexyl, Cycloheptyl bedeuten oder $R^{13}$ und $R^{14}$ zusammen mit dem Stickstoffatom, an das sie gebunden sind, einen 5- bis 7-gliedrigen gesättigten Heterocyclus mit 1 oder 2 gleichen oder verschiedenen Heteroatomen, vorzugsweise mit den Heteroatomen Stickstoff oder Sauerstoff oder beiden Atomen, bilden, insbesondere Pyrrolidin, Piperidin oder Morpholin, in Gegenwart einer unter den Reaktionsbedingungen Ammoniakfreisetzenden Verbindung, wie Ammoniumformiat, -acetat, -chlorid, Formamid oder Acetamid, bevorzugt Ammoniumacetat und Formamid, gegebenenfalls in einem Lösungsmittel, wie Acetonitril, Dimethylformamid, Dimethylacetamid, N-Methylpyrrolidon, z. B. bei Temperaturen zwischen 20 °C und der Siedetemperatur der Lösungsmittels, bevorzugt bei der Siedetemperatur des Lösungsmittels, umsetzt.

Verbindungen der Formel II sind bekannt bzw. lassen sich auf bekannte Weise darstellen (z. B. J. Org. Chem. 50 (1985) 2407, Chem. Ber. 124 (1991) 571).

Verbindungen der Formel III sind bekannt bzw. lassen sich auf bekannte Weise darstellen (z. B. Chem. Ber. 124 (1991) 571).

Die Verbindungen der Formel I und ihre pflanzenverträglichen Säureadditionssalze und Metallsalzkomplexe eignen sich als Fungizide und Nematizide.

Herstellungsbeispiel

2-Benzyl-5,6-dihydro-3-phenyl[1,10]phenanthrolin (Verb. 51)

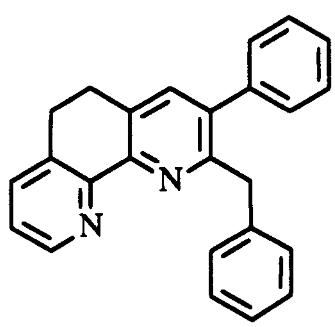

Eine Lösung von 1,47 g (10 mmol) 6,7-Dihydro-5H-chinolinon in 40ml wasserfreiem N,N-Dimethylformamid wird auf 160 °C erhitzt (Argonatmosphäre). Über einen Zeitraum von 60 min wird eine Suspension von 3,34 g (11 mmol) 4-Dimethylamino-1,3-diphenyl-2-butanon-Hydrochlorid und 3,00 g (39 mmol) Ammoniumacetat in 25 ml trockenem N,N-Dimethylformamid zugetropft. Man beläßt die Reaktionsmischung weitere 2 h auf 160 °C und entfernt dann das Lösungsmittel am Rotationsverdampfer. Der Rückstand wird mit 20 ml Wasser, dann mit konz. Ammoniak bis zur deutlich alkalischen Reaktion versetzt ung viermal mit je 30ml Dichlormethan extrahiert. Die vereinigten organischen Phasen werden mit Wasser neutral gewaschen, über Natriumsulfat getrocknet und das Lösungsmittel wird am Rotationsverdampfer entfernt. Das Produkt wird

aus dem Rückstand mittels Säulenchromatographie (Aluminiumoxid, 6 % Wasser = Aktivität III / Dichlormethan) isoliert.
Ausbeute: 1,20 g (34 %), gelbe Kristalle;
Schmp.: 111 ° C (Petrolether 100-140)
In entsprechender Weise können die in der folgenden Tabelle beschriebenen Verbindungen hergestellt werden.

Tabelle 1

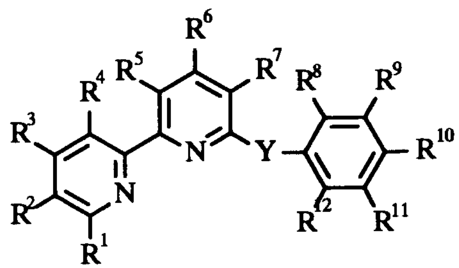

I

| Verb. Nr. | $R^1$ | $R^2$ | $R^3$ | $R^4$ $R^5$ | $R^6$ | $R^7$ $R^8$ | $R^9$ | $R^{10}$ | $R^{11}$ | $R^{12}$ | Y | Fp/IR (Film) [$cm^{-1}$] |
|---|---|---|---|---|---|---|---|---|---|---|---|---|
| 1 | H | H | H | $-CH_2-$ | H | $-CH_2-$ | H | H | H | H | Einfachbindung | 225-231°C (Zers.)*) |
| 2 | H | H | H | $-CH_2-$ | H | $-CH_2-$ | $CH_3$ | H | H | H | Einfachbindung | |
| 3 | H | H | H | $-CH_2-$ | H | $-CH_2-$ | H | $OCH_3$ | H | H | Einfachbindung | |
| 4 | H | H | H | $-CH_2-$ | H | $-(CH_2)_2-$ | H | H | H | H | Einfachbindung | 187-190°C (Zers.)*) |
| 5 | H | H | H | $-CH_2-$ | H | $-(CH_2)_2-$ | H | t-Bu | H | H | Einfachbindung | |
| 6 | H | H | H | $-CH_2-$ | H | $-(CH_2)_3-$ | H | H | H | H | Einfachbindung | |
| 7 | H | H | H | $-(CH_2)_2-$ | H | $-CH_2-$ | H | H | H | H | Einfachbindung | 206°C*) |
| 8 | H | H | H | $-(CH_2)_2-$ | H | $-CH_2-$ | H | H | Ph | H | Einachbindung | |
| 9 | H | H | H | $-(CH_2)_2-$ | H | $-(CH_2)_2-$ | H | H | H | H | Einfachbindung | 183°C*) |

| Verb. Nr. | $R^1$ | $R^2$ | $R^3$ | $R^4$ $R^5$ | $R^6$ | $R^7$ $R^8$ | $R^9$ | $R^{10}$ | $R^{11}$ | $R^{12}$ | Y | Fp/IR (Film) [$cm^{-1}$] |
|---|---|---|---|---|---|---|---|---|---|---|---|---|
| 10 | H | H | H | $-(CH_2)_2-$ | H | $-(CH_2)_2-$ | H | $OCH_3$ | H | H | Einfachbindung | 120°C |
| 11 | H | H | H | $-(CH_2)_2-$ | H | $-(CH_2)_2-$ | H | Cl | H | H | Einfachbindung | |
| 12 | H | H | H | $-(CH_2)_2-$ | H | $-(CH_2)_2-$ | H | H | Ph | H | Einfachbindung | |
| 13 | H | H | H | $-(CH_2)_2-$ | H | $-(CH_2)_2-$ | H | H | $NO_2$ | H | Einfachbindung | 245°C |
| 14 | H | H | H | $-(CH_2)_2-$ | H | $-(CH_2)_3-$ | H | H | H | H | Einfachbindung | 174°C |
| 15 | H | H | H | $-(CH_2)_2-$ | H | $-(CH_2)_3-$ | H | H | $OCH_3$ | H | Einfachbindung | |
| 16 | H | H | H | $-(CH_2)_2-$ | H | $-(CH_2)_4-$ | H | H | H | H | Einfachbindung | |
| 17 | H | H | H | $-(CH_2)_3-$ | H | $-CH_2-$ | H | H | H | H | Einfachbindung | |
| 18 | H | H | H | $-(CH_2)_3-$ | H | $-(CH_2)_2-$ | H | H | H | H | Einfachbindung | |
| 19 | H | H | H | $-(CH_2)_3-$ | H | $-(CH_2)_3-$ | H | H | H | H | Einfachbindung | 273°C |
| 20 | H | H | H | $-(CH_2)_3-$ | H | $-(CH_2)_3-$ | H | $CH_3$ | H | H | Einfachbindung | |
| 21 | H | H | H | $-(CH_2)_3-$ | H | $-(CH_2)_4-$ | H | H | H | H | Einfachbindung | |
| 22 | H | H | H | $-(CH_2)_4-$ | H | $-(CH_2)_2-$ | H | H | H | H | Einfachbindung | |
| 23 | H | H | H | $-(CH_2)_4-$ | H | $-(CH_2)_3-$ | H | H | H | H | Einfachbindung | |
| 24 | H | H | H | $-(CH_2)_4-$ | H | $-(CH_2)_4-$ | H | H | H | H | Einfachbindung | |
| 25 | Ph | H | H | $-CH_2-$ | H | $-(CH_2)_2-$ | H | H | H | H | Einfachbindung | |
| 26 | $CH_3$ | H | H | $-CH_2-$ | H | $-(CH_2)_2-$ | H | H | Ph | H | Einfachbindung | |
| 27 | $CH_3$ | $CH_3$ | H | $-(CH_2)_2-$ | H | $-CH_2-$ | $CH_3$ | H | H | H | Einfachbindung | |
| 28 | Ph | H | Ph | $-(CH_2)_2-$ | H | $-(CH_2)_2-$ | H | H | H | H | Einfachbindung | |
| 29 | Cl | $CH_3$ | H | $-(CH_2)_2-$ | H | $-(CH_2)_3-$ | H | $CH_3$ | H | H | Einfachbindung | |

| Verb. Nr. | $R^1$ | $R^2$ | $R^3$ | $R^4$ $R^5$ | $R^6$ | $R^7$ | $R^8$ | $R^9$ | $R^{10}$ | $R^{11}$ | $R^{12}$ | Y | Fp/IR (Film) [$cm^{-1}$] |
|---|---|---|---|---|---|---|---|---|---|---|---|---|---|
| 30 | H | H | H | $-CH_2-$ | H | H | H | H | H | H | H | Einfachbindung | |
| 31 | H | H | H | $-CH_2-$ | H | $CH_3$ | H | H | H | H | H | Einfachbindung | 160°C*) |
| 32 | H | H | H | $-CH_2-$ | H | Ph | H | H | H | H | H | Einfachbindung | 203-210°C (Zers.)*) |
| 33 | H | H | H | $-CH_2-$ | H | $PhCH_2-$ | H | H | H | H | H | Einfachbindung | |
| 34 | H | H | H | $-(CH_2)_2-$ | H | H | H | H | H | H | H | Einfachbindung | |
| 35 | H | H | H | $-(CH_2)_2-$ | H | $CH_3$ | H | H | H | H | H | Einfachbindung | |
| 36 | H | H | H | $-(CH_2)_2-$ | H | Ph | H | H | H | H | H | Einfachbindung | 162°C*) |
| 37 | H | H | H | $-(CH_2)_2-$ | H | $PhCH_2-$ | H | H | H | H | H | Einfachbindung | 214°C |
| 38 | H | H | H | $-(CH_2)_3-$ | H | H | H | H | H | H | H | Einfachbindung | |
| 39 | H | H | H | $-(CH_2)_3-$ | H | $CH_3$ | H | H | H | H | H | Einfachbindung | |
| 40 | H | H | H | $-(CH_2)_3-$ | H | Ph | H | H | H | H | H | Einfachbindung | |
| 41 | H | H | H | $-(CH_2)_3-$ | H | $PhCH_2-$ | H | H | H | H | H | Einfachbindung | |
| 42 | H | H | H | $-(CH_2)_4-$ | H | H | H | H | H | H | H | Einfachbindung | |
| 43 | H | H | H | $-(CH_2)_4-$ | H | $CH_3$ | H | H | H | H | H | Einfachbindung | |
| 44 | H | H | H | $-(CH_2)_4-$ | H | Ph | H | H | H | H | H | Einfachbindung | |
| 45 | H | H | H | $-(CH_2)_4-$ | H | $PhCH_2-$ | H | H | H | H | H | Einfachbindung | |
| 46 | Ph | H | H | $-CH_2-$ | H | $CH_3$ | H | H | $CH_3$ | H | H | Einfachbindung | |
| 47 | H | H | H | $-CH_2-$ | H | 4-Cl-Ph | H | H | Cl | H | H | Einfachbindung | |
| 48 | $CH_3$ | $CH_3$ | H | $-(CH_2)_2-$ | H | Ph | H | H | $CH_3$ | H | H | Einfachbindung | |

| Verb. Nr. | $R^1$ | $R^2$ | $R^3$ | $R^4$ $R^5$ | $R^6$ | $R^7$ | $R^8$ | $R^9$ | $R^{10}$ | $R^{11}$ | $R^{12}$ | Y | Fp/IR (Film) [$cm^{-1}$] |
|---|---|---|---|---|---|---|---|---|---|---|---|---|---|
| 49 | Ph | H | H | $-(CH_2)_2-$ | H | $4-CH_3-PhCH_2-$ | H | H | $CH_3$ | H | H | Einfachbindung | |
| 50 | H | H | H | $-CH_2-$ | H | Ph | H | H | H | H | H | $-CH_2-$ | |
| 51 | H | H | H | $-(CH_2)_2-$ | H | Ph | H | H | H | H | H | $-CH_2-$ | 111°C |
| 52 | H | H | H | $-(CH_2)_3-$ | H | Ph | H | H | H | H | H | $-CH_2-$ | |
| 53 | $CH_3$ | $CH_3$ | H | $-(CH_2)_2-$ | H | $4-CH_3-Ph$ | H | H | $CH_3$ | H | H | $-CH_2-$ | |
| 54 | Ph | H | H | $-(CH_2)_2-$ | H | $4-OCH_3-Ph$ | H | H | $OCH_3$ | H | H | $-CH_2-$ | |
| 55 | Ph | H | H | $-(CH_2)_2-$ | H | 4-Cl-Ph | H | H | Cl | H | H | $-CH_2-$ | |

*) bekannt aus: Chem. Ber. **124** (1991) 571

Die Verbindungen eignen sich als Fungizide und Nematizide.

Die Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zübereitungen sind:

I. eine Lösung aus 90 Gew.-Teilen der Verbindung 10 und 10 Gew.-Teilen N-Methyl-a-pyrrolidon, die zur Anwendung in Form kleinster Tropfen geeignet ist;

II. eine Mischung aus 20 Gew.-Teilen der Verbindung 13, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung 14, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung 19, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung 37, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung 51 und 97 Gew.-Teilen feinteiligem Kaolin; dieses Stäubemittel enthält 3 Gew.-% Wirkstoff;

VII. eine innige Mischung aus 30 Gew.-Teilen der Verbindung 10, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung 13, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung 14, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls.

Die Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgenden Anwendungsbeispiele zeigen die gute fungizide Wirkung der Verbindungen.

Anwendungsbeispiel 1

Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensübstanz enthielt, besprüht. Um die Wirkungsdauer der Wirkstoffe beurteilen zu können, wurden die Pflanzen nach dem Antrocknen des Spritzbelages 8 Tage mit Gewächshaus aufgestellt. Erst dann wurden die Blätter mit einer Zoosporenaufschwemmung von Plasmopara viticola (Rebenperonospora) infiziert. Danach wurden die Reben zunächst 48 Stunden in einer wasserdampfgesättigten Kammer bei 24 °C und anschließend für 5 Tage in einem Gewächshaus bei Temperaturen zwischen

20 und 30 °C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruchs auf den Blattunterseiten.

Das Ergebnis des Versuchs zeigt, daß bei der Anwendung einer 250 ppm Wirkstoff enthaltenden Spritzbrühe die Verbindungen 4, 7, 19, 9, 10, 13, 14, 32, 37 und 51 eine sehr gute fungizide Wirkung zeigen (10 % Befall).

Anwendungsbeispiel 2

Wirksamkeit gegen Botrytis cinerea

Paprikasämlinge der Sorte "Neusiedler Ideal Elite" wurden, nachdem sich 4 - 5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 - 24 °C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedecken.

Das Ergebnis des Versuchs zeigt, daß bei Anwendung einer 500 ppm Wirkstoff enthaltenden Spritzbrühe die Verbindungen Nr. 7, 9, 10, 14, 36 und 37 eine sehr gute fungizide Wirkung zeigen (10 % Befall).

## Patentansprüche

**1.** Verwendung von substituierten Dipyridin-Derivaten der allgemeinen Formel I,

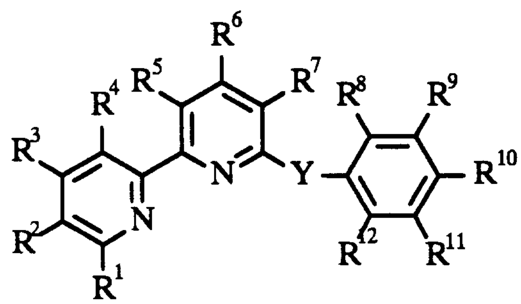

I

worin

| | |
|---|---|
| $R^1$,$R^2$,$R^3$ = | unabhängig voneinander Wasserstoff, $C_1$-$C_4$-Alkyl, Phenyl, Halogen, |
| $R^4$ und $R^5$ | zusammen eine Polymethylenkette der Formel - $(CH_2)_m$-mit m = 1 bis 4 bilden, |
| $R^6$ = | Wasserstoff, |
| $R^7$ = | Wasserstoff, $C_1$-$C_4$-Alkyl, Phenyl-$C_1$-$C_2$-alkyl, Phenyl, wobei die beiden letztgenannten Reste im Phenylteil bis zu dreifach durch Halogen, $C_1$-$C_4$-Alkyl, $C_1$-$C_4$-Alkoxy oder $C_1$-$C_4$-Haloalkyl substituiert sein können, |
| $R^8$ = | Wasserstoff, $C_1$-$C_4$-Alkyl, $C_1$-$C_4$-Alkoxy, $C_1$-$C_4$-Haloalkyl, Halogen oder |
| $R^7$ und $R^8$ | können, für den Fall Y = Einfachbindung, zusätzlich zusammen eine Polymethylenkette der Formel $-(CH_2)_n-$ mit n = 1 bis 4 bilden, |
| $R^9$,$R^{10}$,$R^{11}$, $R^{12}$ = | unabhängig voneinander Wasserstoff, $C_1$-$C_4$-Alkyl, $C_1$-$C_4$-Alkoxy, $C_1$-$C_4$-Haloalkyl, Phenyl, Halogen, Nitro, |
| Y = | eine Einfachbindung oder $-CH_2-$, |

bedeuten, sowie deren pflanzenverträglichen Säureadditionssalze und Metallsalzkomplexe zur Bekämpfung von Schädlingen.

**2.** Substituierte Dipyridin-Derivate der allgemeinen Formel I gemäß Anspruch 1, sowie deren pflanzenverträgliche Säureadditionssalze und Metallsalzkomplexe, ausgenommen die Verbindungen der Formel I, in der die Reste $R^1$, $R^2$, $R^3$, $R^6$, $R^9$, $R^{10}$, $R^{11}$ und $R^{12}$ Wasserstoff und Y eine Einfachbindung bedeuten und $R^4$, $R^5$, $R^7$ und $R^8$ die folgenden Bedeutungen haben:

| $R^4$, $R^5$ | $R^7$ | $R^8$ |
|---|---|---|
| $-CH_2-$ | $-CH_2-$ | |
| $-CH_2-$ | $-(CH_2)_2-$ | |
| $-(CH_2)_2-$ | $-CH_2-$ | |
| $-(CH_2)_2-$ | $-(CH_2)_2-$ | |
| $-CH_2-$ | $CH_3$ | H |
| $-CH_2-$ | Ph | H |
| $-(CH_2)_2-$ | Ph | H |

**3.** Verfahren zur Herstellung von substituierten Dipyridin-Derivaten der Formel I gemäß Anspruch 2, dadurch gekennzeichnet, daß man eine Verbindung der Formel II,

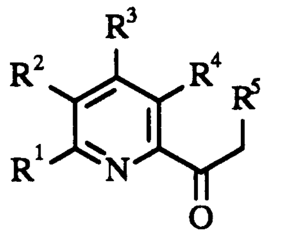

II

worin $R^1$ bis $R^5$ die Bedeutung wie in Anspruch 2 besitzen, mit einer Verbindung der Formel III,

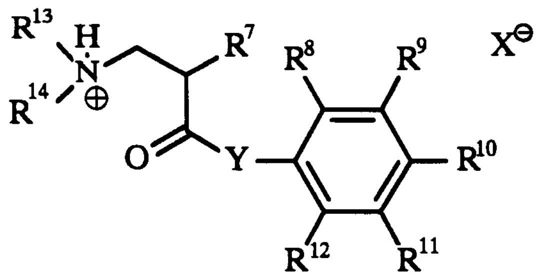

III

worin $R^7$ bis $R^{12}$ und Y die Bedeutung wie in Anspruch 2 besitzen, X das Anion einer organischen oder anorganischen Säure und $R^{13}$ und $R^{14}$ unabhängig voneinander $C_1$-$C_4$-Alkyl, $C_5$-$C_7$-Cycloalkyl bedeuten oder $R^{13}$ und $R^{14}$ zusammen mit dem Stickstoffatom, an das sie gebunden sind, einen 5-7-gliedrigen gesattigten Heterocyclus mit 1 oder 2 gleichen oder verschiedenen Heteroatomen, vorzugsweise mit den Heteroatomen Stickstoff oder Sauerstoff oder beiden Atomen bilden, in Gegenwart einer Ammoniak-freisetzenden Verbindung umsetzt.

**4.** Schädlingsbekämpfungsmittel, enthaltend einen flüssigen oder festen Trägerstoff und ein substituiertes Dipyridin-Derivat der Formel I gemäß Anspruch 1.

**5.** Verfahren zur Bekämpfung von Schädlingen, dadurch gekennzeichnet, daß man eine wirksame Menge eines substituierten Dipyridin-Derivats der Formel I gemäß Anspruch 1 auf Schädlinge, insbesondere Pilze und Nematoden, oder deren Lebensraum, den Boden oder auf Saatgüter einwirken läßt.

**6.** Verbindung der Formel I gemäß Anspruch 2, in der $R^1$, $R^2$, $R^3$, $R^6$, $R^9$, $R^{11}$ und $R^{12}$ Wasserstoff, $R^4$ und $R^5$ $(CH_2)_2$, $R^7$ und $R^8$ $(CH_2)_2$, $R^{10}$ Methoxy und Y eine Einfachbindung bedeuten.

**7.** Verbindung der Formel I gemäß Anspruch 2, in der $R^1$, $R^2$, $R^3$, $R^6$, $R^9$, $R^{10}$ und $R^{12}$ Wasserstoff, $R^4$ und $R^5$ $(CH_2)_2$, $R^7$ und $R^8$ $(CH_2)_2$, $R^{11}$ $NO_2$ und Y eine Einfachbindung bedeuten.

**8.** Verbindung der Formel I gemäß Anspruch 2, in der $R^1$, $R^2$, $R^3$, $R^6$, $R^8$, $R^9$, $R^{10}$, $R^{11}$ und $R^{12}$ Wasserstoff, $R^4$ und $R^5$ $(CH_2)_2$, $R^7$ Phenyl und Y $CH_2$ bedeuten.

Europäisches Patentamt

# EUROPÄISCHER RECHERCHENBERICHT

Nummer der Anmeldung

EP 93 12 0038

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | CHEM. BER.<br>Bd. 124, Nr. 3, 1991,<br>Seiten 571 - 576<br>WESTERWELLE, U. ET AL 'beta-amino ketones as key intermediates in the synthesis of pyridines'<br>--- | 2-3 | C07D471/04<br>A01N43/90<br>//(C07D471/04,221:00,221:00) |
| A | EP-A-0 334 148 (BAYER AG)<br>27. September 1989<br>* das ganze Dokument *<br>--- | 1-8 | |
| A | EP-A-0 363 643 (BAYER AG)<br>18. April 1990<br>* das ganze Dokument *<br>--- | 1-8 | |
| A | EP-A-0 282 893 (BAYER AG)<br>21. September 1988<br>* das ganze Dokument *<br>----- | 1-8 | |

RECHERCHIERTE SACHGEBIETE (Int. Cl.5)

C07D
A01N

Der vorliegende Recherchenbericht wurde für alle Patentansprüche erstellt

| Recherchenort | Abschlußdatum der Recherche | Prüfer |
|---|---|---|
| MUENCHEN | 03 MAERZ 1994 | SCRUTON-EVANS I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)